# EUROPÄISCHE PATENTSCHRIFT

(11) **EP 2 441 743 B1**
(45) Veröffentlichungstag und Bekanntmachung des Hinweises auf die Patenterteilung: **21.08.2013**
(21) Anmeldenummer: 11007597.5
(22) Anmeldetag: 19.09.2011
(51) Int. Cl.: C07C 17/263, C07C 25/18

(54) **Verfahren zur Herstellung von aromatischen Verbindungen**
Method for manufacturing aromatic compounds
Procédé destiné à la production de composés aromatiques

(30) Priorität: 14.10.2010 DE 102010048499
(43) Veröffentlichungstag der Anmeldung: 18.04.2012
(73) Patentinhaber: Merck Patent GmbH, 64293 Darmstadt (DE)
(72) Erfinder: Cezanne, Carina, 64331 Weiterstadt (DE); Wekwert, Volker, 64372 Ober-Ramstadt (DE)

(56) Entgegenhaltungen:
- EP-A1- 1 900 792
- DE-A1-102008 036 807

## Beschreibung

Die vorliegende Erfindung betrifft ein Verfahren zur Herstellung von aromatischen Verbindungen durch Kreuzkupplung von aromatischen Borverbindungen mit Halogenaromaten.

Wichtige aromatische Verbindungen werden durch Kreuzkupplungen hergestellt, bei denen C-C-Bindungen zwischen aromatischen Gruppen gebildet werden. Viele der dadurch erhaltenen Aromaten und insbesondere der Heteroaromaten sind von großem Interesse sowohl für die Agro- und Pharmaindustrie als auch für die Materialwissenschaften, insbesondere zur Herstellung von mesogenen Verbindungen. Zu den bevorzugten Umsetzungen dieses Typs zählt die von Suzuki vorgeschlagene Umsetzung von aromatischen Borverbindungen mit Halogenaromaten in Gegenwart einer Base.

Beispielsweise werden diese Reaktionen zur Herstellung von aromatischen oder heteroaromatischen Verbindungen in den Druckschriften WO 2005/123878, WO 2007/079842 und WO 2008/016166 dargelegt. Nachteilig an diesen Reaktionen ist ein relativ hoher Reinigungsaufwand der erhaltenen Reaktionsprodukte. Hierbei ist insbesondere eine Bildung von Dimeren störend, die von den jeweils eingesetzten aromatischen Borverbindungen abgeleitet sind, da diese nur sehr aufwendig abgetrennt werden können. Zum Erhalt von aromatischen Verbindungen mit einer geforderten Reinheit von mindestens 99,5% müssen chromatographische Trennmethoden mehrfach durchgeführt werden.

In den Druckschriften EP 1900792 A1 und DE 102008036807 A1 werden Suzuki-Kopplungen von Phenylboronsäure-Derivaten offenbart. Die Reaktionsgemische enthalten THF, Wasser, Natrium-metaborat und weitere Reagenzien.

Die Bildung von Dimeren der zuvor genannten Art stellt ein bekanntes Problem der zuvor näher ausgeführten Suzuki-Reaktion dar. Dies wird beispielsweise von Miller W.D., et al. in Organic Process Research & Development, 2007, 11, 359-364 ausführlich dargelegt. Als Lösung dieser Problematik wird vorgeschlagen, die Umsetzung unter Ausschluss von Sauerstoff durchzuführen. Hierzu wird ein mildes Reduktionsmittel eingesetzt und die Zugabestelle des Initiators mit Stickstoff gespült. Die vorgeschlagene Arbeitsweise führt gemäß dieser Druckschrift zwar zu einer spürbaren Minimierung der Dimerisation der eingesetzten Borverbindung.

Allerdings ist diese Arbeitsweise sehr aufwendig, wobei die genannte Problematik im Zusammenhang mit der Herstellung von Pharmazeutika beschrieben wurde. Weiterhin führt die in diesem Dokument vorgeschlagene Vorgehensweise insbesondere bei einer Herstellung von aromatischen Verbindungen gemäß den hier vorgestellten Verbindungsklassen nicht zu einem optimalen Ergebnis.

Die vorliegende Erfindung stellt sich daher die Aufgabe, ein Verfahren zur Herstellung von aromatischen Verbindungen, insbesondere durch Umsetzung von aromatischen Borverbindungen mit Halogenaromaten in Gegenwart einer Base, bereitzustellen, welches das gewünschte Produkt in hoher Ausbeute und Reinheit ergibt und vorzugsweise die genannten Nachteile der bekannten Verfahren vermeidet.

Insbesondere sollte das Verfahren zu einer geringen Bildung an Dimeren führen. Weiterhin sollte die erhaltene Reaktionsmischung einfach und kostengünstig aufgereinigt werden können. Darüber hinaus sollte die gewünschten Produkte mit hohen Ausbeuten erhalten werden.

Überraschenderweise wurde gefunden, dass diese sowie weitere nicht explizit genannte Aufgaben, die jedoch aus den hierin einleitend diskutierten Zusammenhängen ohne Weiteres ableitbar oder erschließbar sind, durch Verfahren zur Herstellung von aromatischen Verbindungen mit allen Merkmalen des Patentanspruchs 1 gelöst werden. Zweckmäßige Abwandlungen der erfindungsgemäßen Verfahren werden in den auf Anspruch 1 rückbezogenen abhängigen Ansprüchen unter Schutz gestellt.

Gegenstand der vorliegenden Erfindung ist dementsprechend ein Verfahren zur Herstellung von aromatischen Verbindungen der Formel (I)

Ar¹-Ar² (1),

worin die Reste Ar¹ und Ar² jeweils unabhängig ein optional substituiertes aromatisches oder heteroaromatisches Ringsystem darstellen, umfassend die Umsetzung einer aromatischen Borverbindung der Formel (II) 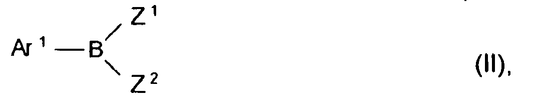 worin Ar¹ die zuvor genannte Bedeutung hat und die Reste Z¹ und Z² unabhängig Hydroxy, Dialkylamino, C₁ - C₁₅ Alkyloxy oder C₆ - C₁₅ Aryloxy darstellen, wobei die beiden Reste Z¹ und Z² auch ein Ringsystem bilden können,
mit einem aromatischen Edukt der Formel (III)

Ar²-Y (III),

worin Ar² die zuvor dargelegte Bedeutung hat und Y ein Halogen, Arylsulfonyloxy, Alkylsulfonyloxy, Perfluoralkylsulfonyloxy, Alkylcarbonyloxy, Arylcarbonyloxy, Alkyloxycarbonyloxy oder Aryloxycarbonyloxy darstellt, unter Verwendung eines Lösungsmittels in Gegenwart einer Base, welches dadurch gekennzeichnet ist, dass ein wasserarmes polares Lösungsmittel, das eine Polaritätskonstante E_{T} (30) > 175 kJ/mol und/oder eine Dielektrizitätskonstante εᵣ >15 aufweist, und ein basisches Borat, vorzugsweise ein Alkaliborat als Base zur Umsetzung eingesetzt werden, wobei der Wassergehalt der Reaktionsmischung höchstens 5 Gew.-% beträgt.

Die nach dem erfindungsgemäßen Verfahren zugänglichen Verbindungen werden in außerordentlich hohen Reinheiten des Rohgemisches von in der Regel größer als 95 %, vorzugsweise größer als 97% erhalten. Der Gehalt an Dimer (Nebenprodukt) liegt dabei vorzugsweise unter 0,5%. Das erfindungsgemäße Verfahren vermindert den bislang hohen notwendigen Aufwand zur Aufreinigung des Rohreaktionsprodukts. Insbesondere kann ein für viele Zwecke ausreichend reines Endprodukt ohne mehrfache Durchführung von chromatographischen Trennverfahren erhalten werden.

Zugleich wird eine sehr hohe Ausbeute an Reinprodukt erhalten. Weiterhin können die Produkte insgesamt kostengünstig und ohne Umweltgefährdung erhalten werden.

Durch das vorliegende Verfahren sind insbesondere aromatische oder heteroaromatische Verbindungen der Formel (I)

Ar¹-Ar² (I),

worin die Reste Ar¹ und Ar² jeweils unabhängig ein optional substituiertes aromatisches oder heteroaromatisches Ringsystem darstellen, erhältlich.

Ein aromatisches Ringsystem im Sinne der vorliegenden Erfindung enthält 6 bis 60, vorzugsweise 6 bis 40 und besonders bevorzugt 6 bis 20 C-Atome im Ringsystem. Das heteroaromatische Ringsystem im Sinne der vorliegenden Erfindung enthält 2 bis 60, vorzugsweise 2 bis 40 und besonders bevorzugt 2 bis 20 C-Atome und mindestens ein Heteroatom im Ringsystem, mit der Maßgabe, dass die Summe aus C-Atomen und Heteroatomen mindestens 5 ergibt. Die Heteroatome sind vorzugsweise ausgewählt aus Si, N, P, O, S und/oder Se. Unter einem aromatischen oder heteroaromatischen Ringsystem im Sinne der vorliegenden Erfindung soll darüber hinaus ein System verstanden werden, das nicht notwendigerweise nur Aryl- oder Heteroarylgruppen enthält, sondern in dem auch mehrere Aryl- oder Heteroarylgruppen durch eine nichtaromatische Einheit, wie z. B. ein sp³-hybridisiertes C-Atom, ein N- oder 0-Atom, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne der vorliegenden Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Ein erfindungsgemäßes aromatisches oder heteroaromatisches Ringsystem kann beliebige Substituenten aufweisen, vorzugsweise Halogene, Alkylgruppen, (Alkyl-Cycloalkylgruppen und Alkoxygruppen, die wiederum durch Halogen substituiert sein können.

Als aromatisches oder heteroaromatisches Ringsystem mit 5 bis 60 Ringatomen, welches noch jeweils mit beliebigen Resten R (siehe unten) substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin, Benzothiadiazol, Benzanthren, Benzanthracen, Rubicen und Triphenylen.

Der als Substituent der Ringsysteme genannte Rest R bedeutet vorzugsweise eine Gruppe ausgewählt aus Alkyl, Alkenyl, Alkoxy, Cyclohexan und Phenyl, wobei diese Ringe optional durch Alkyl, Cyclohexyl, Alkylcyclohexyl und weiteren Kombinationen daraus substituiert sein können. Bevorzugt bedeutet der Rest R daher Alkyl, 4-Alkylcyclohexan, Alkyl-4,4'-bicyclohexan, 4-Alkylphenyl oder Alkyl-1,1'-Biphenyl, worin Benzolringe durch ein oder mehrere Fluoratome substituiert sein können.

Sofern es sich bei einem Alkylrest um einen gesättigten Rest handelt, wird er auch als "Alkanyl" bezeichnet. Ferner umfasst der Ausdruck "Alkyl" auch unsubstituierte oder entsprechend insbesondere mit F, Cl, Br, l und/oder -CN ein- oder mehrfach gleich oder verschieden substituierte Kohlenwasserstoffreste, in denen eine oder mehrere CH₂-Gruppen derart durch -O- ("Alkoxy", "Oxaalkyl"), -S- ("Thioalkyl"),-SO₂-, -CH=CH-("Alkenyl"), C≡C- ("Alkinyl"), -CO-O- oder -O-CO- ersetzt sein können, dass Heteroatome (O, S) in der Kette nicht direkt miteinander verknüpft sind. Vorzugsweise ist Alkyl ein geradkettiger oder verzweigter, unsubstituierter oder substituierter Alkanyl-, Alkenyl- oder Alkoxyrest mit 1, 2, 3, 4, 5, 6, 7 oder 8 Kohlenstoffatomen. Sofern Alkyl einen Alkanylrest bedeutet, ist dieser bevorzugt Methyl, Ethyl, n-Propyl, i-Propyl, n-Butyl, i-Butyl, t-Butyl, n-Pentyl, Neopentyl, n-Hexyl, n-Heptyl, n-Octyl; CF₃, CHF₂, CH₂F; CF₂CF₃. Besonders bevorzugt ist der Alkanylrest geradkettig und unsubstituiert oder mit F substituiert.

Da in einem Alkylrest eine oder mehrere CH₂-Gruppen durch -O- ersetzt sein können, umfasst der Ausdruck "Alkyl" auch "Alkoxy"- beziehungsweise "Oxaalkyl"-Reste. Unter Alkoxy ist ein O-Alkyl-Rest zu verstehen, in dem das Sauerstoffatom direkt mit der durch den Alkoxyrest substituierten Gruppe oder dem substituierten Ring verbunden ist und Alkyl wie oben definiert ist; vorzugsweise ist Alkyl dann Alkanyl oder Alkenyl. Bevorzugte Alkoxyreste sind Methoxy, Ethoxy, Propoxy, Butoxy, Pentoxy, Hexoxy, Heptoxy und Octoxy, wobei jeder dieser Reste auch substituiert sein kann, und zwar vorzugsweise.mit einem oder mehreren Fluoratomen. Besonders bevorzugt ist Alkoxy -OCH₃, -OC₂H₅, -O-n-C₃H₇, -O-n-C₄H₉, -O-t-C₄H₉, -OCF₃, -OCHF₂, -OCHF oder -OCHFCHF₂. Im Zusammenhang mit der vorliegenden Erfindung bedeutet der Ausdruck "Oxaalkyl" Alkylreste, in denen wenigstens eine nicht-terminale CH₂-Gruppe durch -O- derart ersetzt ist, dass keine benachbarten Heteroatome (O, S) vorliegen. Vorzugsweise umfasst Oxaalkyl geradkettige Reste der Formel CₐH₂ₐ₊₁-O-(CH₂)_{b}-, wobei a und b jeweils unabhängig voneinander 1, 2, 3, 4, 5, 6, 7, 8, 9 oder 10 bedeuten; besonders bevorzugt ist a eine ganze Zahl von 1 bis 6 und b 1 oder 2.

Im Zusammenhang der vorliegenden Erfindung bedeutet der Ausdruck "Alkenyl" einen wie oben definierten Alkylrest, in dem eine oder mehrere -CH=CH-Gruppen vorhanden sind. Sofern zwei -CH=CH-Gruppen in dem Rest vorhanden sind, kann dieser auch als "Alkadienyl" bezeichnet werden. Ein Alkenylrest kann 2 bis 15 (d. h. 2, 3, 4, 5, 6, 7, 8, 9, 10, 11, 12, 13, 14 oder 15) Kohlenstoffatome enthalten und ist verzweigtkettig oder vorzugsweise geradkettig. Der Rest ist unsubstituiert oder ein- oder mehrfach gleich oder verschieden insbesondere mit F, Cl, Br, I und/oder CN substituiert. Ferner können eine oder mehrere CH₂-Gruppen jeweils unabhängig voneinander durch -O-, -S-, -C≡C-, -CO-O-, -OC-O- so ersetzt sein, dass Heteroatome (O, S) nicht direkt miteinander verbunden sind. Falls die CH=CH-Gruppe an beiden Kohlenstoffatomen einen anderen Rest als Wasserstoff trägt, etwa wenn sie eine nicht-terminale Gruppe ist, kann die CH=CH-Gruppe in zwei Konfigurationen vorliegen, nämlich als E-Isomer und als Z-Isomer. Im Allgemeinen ist das E-Isomer (trans) bevorzugt. Vorzugsweise enthält der Alkenylrest 2, 3, 4, 5, 6 oder 7 Kohlenstoffatome und bedeutet Vinyl, 1E-Propenyl, 1E-Butenyl, 1 E-Pentenyl, 1E-Hexenyl, 1E-Heptenyl, 2-Propenyl, 2E-Butenyl, 2E-Pentenyl, 2E-Hexenyl, 2E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl und 6-Heptenyl. Besonders bevorzugte Alkenylreste sind Vinyl, 1 E-Propenyl und 3E-Butenyl.

Falls in einem Alkylrest eine oder mehrere CH₂-Gruppen durch -C≡Cersetzt sind, liegt ein Alkinylrest vor. Auch die Ersetzung von einer oder mehreren CH₂-Gruppen durch -CO-O- or -O-CO- ist möglich. Dabei sind die folgenden dieser Reste bevorzugt: Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 2-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)-ethyl, 3-(Methoxycarbonyl)propyl, 3-(Ethoxy-carbonyl)-propyl oder 4-(Methoxycarbonyl)butyl.

Im Zusammenhang der vorliegenden Erfindung bedeutet der Ausdruck "Cycloalkyl" einen cyclischen aliphatischen (alicyclischen) Rest mit 3 bis 16 Kohlenstoffatomen, der gesättigt oder partiell ungesättigt ist und unsubstituiert oder einfach oder mehrfach mit Halogen, Carboxy, Nitro, Alkanyl, Alkoxy, -NH₂ und/oder mit -N(Alkanyl)₂ substituiert ist, wobei die Mehrfachsubstitution mit dem gleichen oder mit verschiedenen Substituenten erfolgen kann. Bevorzugt ist der Cycloalkylrest unsubstituiert und weist 5, 6 oder 7 Kohlenstoffatome auf. Insbesondere steht Cycloalkyl für einen Cyclohexylrest.

Im Zusammenhang der vorliegenden Erfindung steht "Alkylen" für einen divalenten aliphatischen Kohlenwasserstoffrest mit 1, 2, 3, 4, 5, 6, 7, 8 Kohlenstoffatomen in der Kette, der gegebenenfalls auch einfach oder mehrfach mit Halogen, CN, Carboxy, Nitro, Alkanyl, Alkoxy, -NH₂ oder mit -N(Alkanyl)₂ substituiert sein kann, wobei die Mehrfachsubstitution mit dem gleichen oder mit verschiedenen Substituenten erfolgen kann. Bevorzugt steht "Alkylen" für einen geradkettigen, unsubstituierten oder mit Methyl einfach oder zweifach substituierten, gesättigten aliphatischen Rest mit 1, 2, 3, 4, 5, 6 Kohlenstoffatomen, insbesondere für -CH₂CH₂CH₂- und -CH₂C(CH₃)₂CH₂-.

"Halogen" steht im Zusammenhang der vorliegenden Erfindung für Fluor, Chlor, Brom beziehungsweise lod.

Im Zusammenhang der vorliegenden Erfindung bedeutet der Ausdruck "Aryl" - sofern er nicht an anderer Stelle der Beschreibung oder in den Ansprüchen im Einzelfall abweichend definiert ist - einen aromatischen Kohlenwasserstoff mit 6 bis 14 Kohlenstoffatomen, der gegebenenfalls mit Halogen, Nitro, Alkanyl, Alkoxy, -NH₂ oder mit -N(Alkanyl)₂ einfach oder mehrfach substituiert ist, wobei die Mehrfachsubstitution mit dem gleichen oder mit verschiedenen Substituenten erfolgen kann. Insbesondere steht "Aryl" für einen unsubstituierten oder substituierten Phenyl- oder Naphthylrest.

Im Zusammenhang mit der vorliegenden Erfindung steht der Ausdruck "Aryloxy" für einen Aryl-O-Rest, insbesondere einen Phenyl-O-Rest.

Im Zusammenhang mit der vorliegenden Erfindung steht der Ausdruck "Aralkyl" für einen Aryl-Alkyl-Rest, d. h. für einen Rest, in dem ein Aryl-Substituent über eine Alkylbrücke mit einem Atom, einer Kette, einem weiteren Ring, einem anderen Rest oder einer funktionellen Gruppe verknüpft ist. Bei der Alkylbrücke handelt es sich vorzugsweise um einen gesättigten bivalenten Kohlenwasserstoffrest ("Alkylen"), insbesondere um Methylen (-CH₂-) und Ethylen (-CH₂-CH₂-). Bevorzugte Beispiele eines Aralkylrestes sind Benzyl und Phenethyl. Ein "Aralkyl-O-Rest" ist für die Zwecke der vorliegenden Erfindung ein Aralkylrest, der über ein an die Alkylbrücke gebundenes Sauerstoffatom mit einem weiteren Atom, einer Kette, einem anderen Rest oder einer funktionellen Gruppe verknüpft ist. Bevorzugte Beispiele eines Aralkyl-O-Restes sind O-Benzyl und 0-CH₂CH₂Phenyl.

Das erfindungsgemäße Verfahren eignet sich insbesondere zur Herstellung von mesogenen Verbindungen, die zwei direkt verbundene aromatische Ringsysteme enthalten, insbesondere die zwei benachbarte substituierte Phenylringe enthalten. Derartige Verbindungen sind unter anderem in der Druckschriften WO 2005/123878 A1 und US 6,695,979 B2, sowie in der darin zitierten Literatur beschrieben.

Besonders bevorzugte Verbindungen, die durch das vorliegende Verfahren erhältlich sind, entsprechen vorzugsweise der Formel (la) 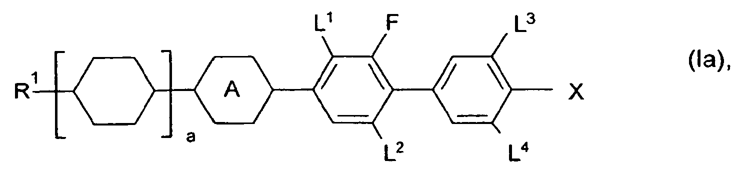 worin
- R¹: einen Alkylrest mit 1 bis 15, vorzugsweise 1 bis 9 Kohlenstoffatomen, bevorzugt einen Alkanyl-, Alkenyl- oder Alkoxyrest mit bis zu 9 Kohlenstoffatomen, der substituiert oder unsubstituiert sein kann,
- a: 0 oder 1

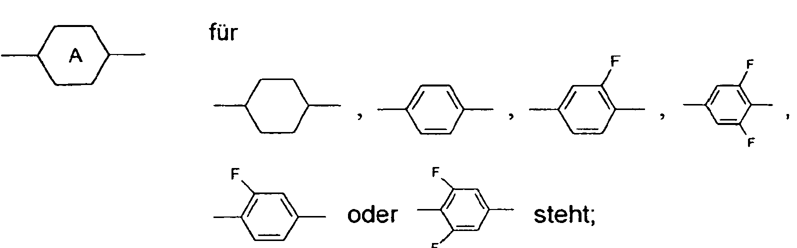

- L¹, L², L³ und L⁴: unabhängig voneinander Wasserstoff oder Fluor
- X: F, Cl, CN, SF₅, SCN, NCS, halogenierter Alkylrest, bevorzugt halogenierter Alkanylrest, halogenierter Alkenylrest, halogenierter Alkoxyrest oder halogenierter Alkenyloxyrest mit bis zu 6 C-Atomen,
bedeuten.

Zur Herstellung der genannten Verbindungen werden aromatische oder heteroaromatische Borverbindungen der Formel (II) 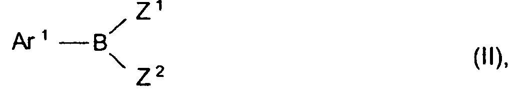 worin Ar¹ die zuvor genannte Bedeutung hat und die Reste Z¹ und Z² unabhängig Hydroxy, Dialkylamino, C₁―C₁₅ Alkyloxy oder C₆―C₁₅ Aryloxy darstellen, wobei die beiden Reste Z¹ und Z² auch ein Ringsystem bilden können, eingesetzt.

Überraschende Vorteile der vorliegenden Erfindung ergeben sich insbesondere bei Verwendung von aromatischen Borverbindungen der Formel (IIa) 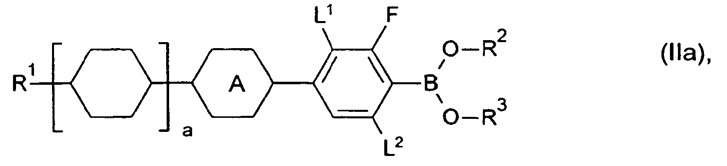 worin
- R¹: einen Alkylrest mit 1 bis 15, vorzugsweise 1 bis 9 Kohlenstoffatomen, bevorzugt einen Alkanyl-, Alkenyl- oder Alkoxyrest mit bis zu 9 Kohlenstoffatomen, der substituiert oder unsubstituiert sein kann, bedeutet;
- R² und R³: jeweils unabhängig Wasserstoff oder einen Alkylrest mit 1 bis 15, vorzugsweise 1 bis 9 Kohlenstoffatomen, bevorzugt einen Alkanyl- oder Alkenylrest, der substituiert oder unsubstituiert sein kann, wobei R² und R³ zusammen auch einen Rest -(CH₂)ₚ- mit p = 2 bis 4 oder 1,2-Phenylen bedeutet, wobei diese Reste optional ein- oder mehrfach durch n-Alkanylreste mit 1-9 C-Atomen substituiert sein können, besonders bevorzugt Wasserstoff bedeuten;

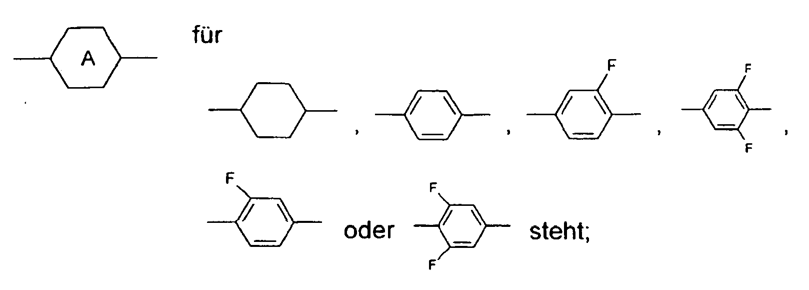

- a: 0 oder 1 bedeutet; und
- L¹ und L²: unabhängig voneinander Wasserstoff oder Fluor bedeuten.
- R¹: bedeutet in den Formeln (la) und (IIa) bevorzugt einen unsubstituierten Alkylrest mit 1 bis 15 C-Atomen, wobei in diesem Rest auch eine oder mehrere CH₂-Gruppen jeweils unabhängig voneinander durch -C≡C-, -CH=CH-, -CF=CF-, -CF=CH-, -CH=CF- oder -O- so ersetzt sein können, dass O-Atome nicht direkt miteinander verknüpft sind.

Bevorzugte aromatische Borverbindungen zur Durchführung des erfindungsgemäßen Verfahrens sind unter anderem

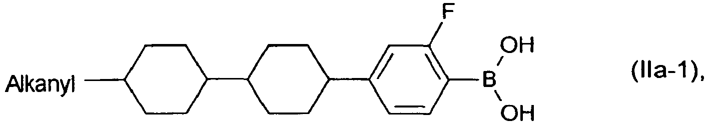

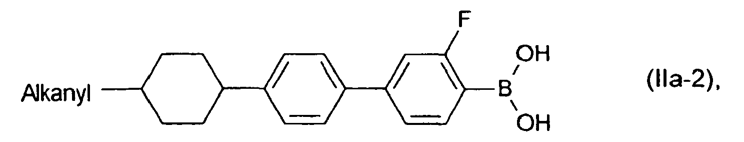

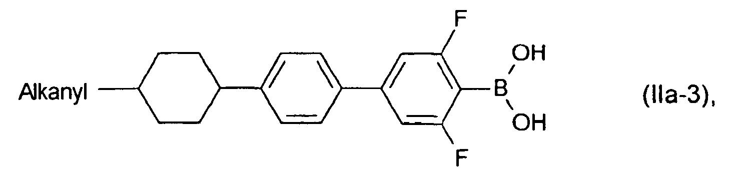

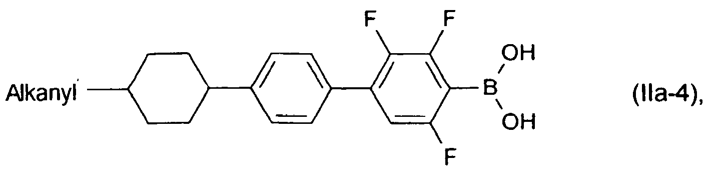

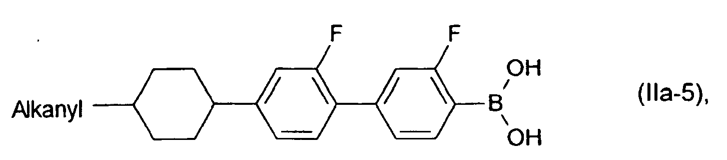

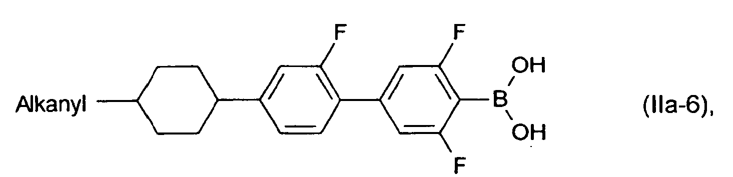

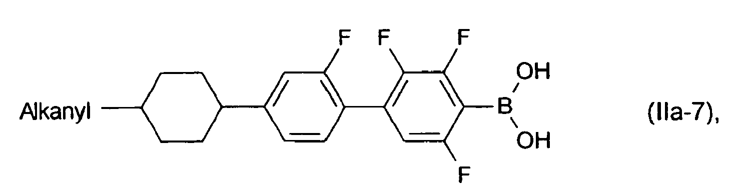

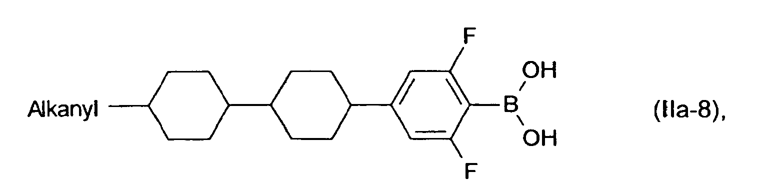

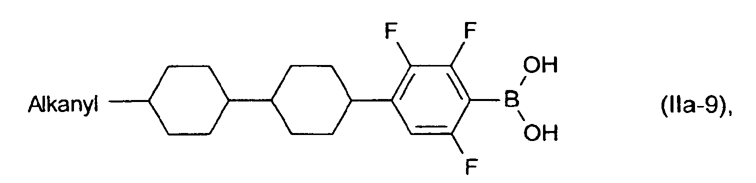

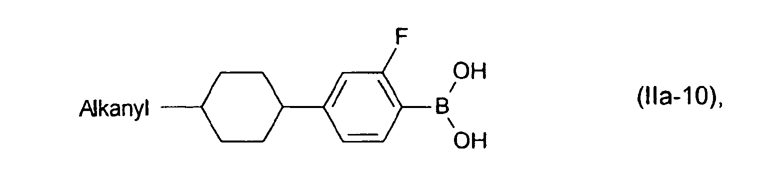

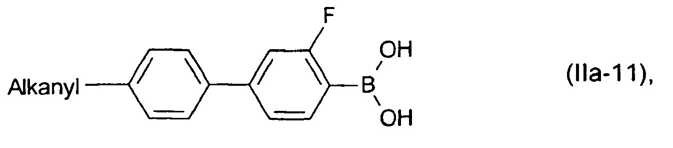

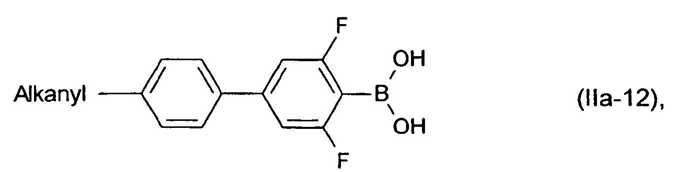

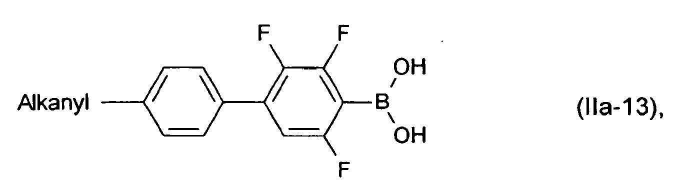

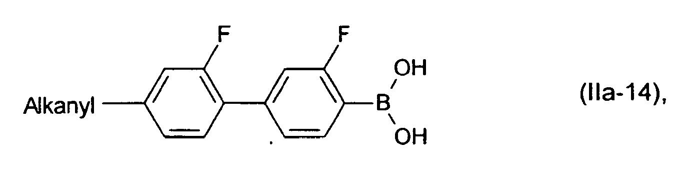

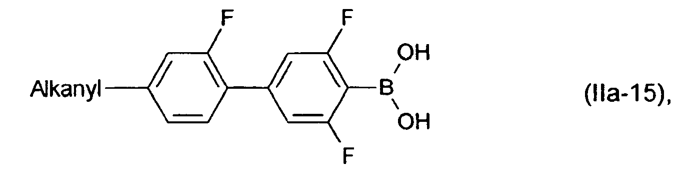

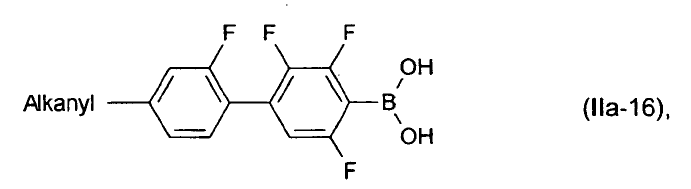

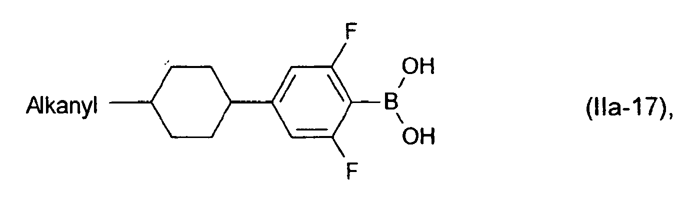

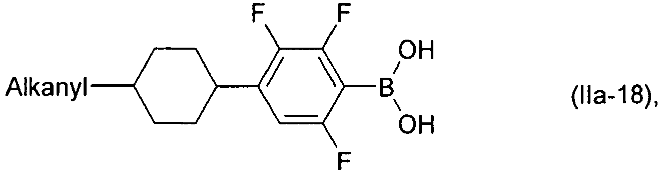

Die Alkanyl-Reste der Verbindungen gemäß der Formeln (IIa-1) bis (IIa-18) weisen bevorzugt 1 bis 15, insbesondere 1 bis 10 und besonders bevorzugt 1 bis 8 Kohlenstoffatome auf, wobei diese Reste bevorzugt linear sind.

Die zuvor dargelegten aromatischen oder heteroaromatischen Borverbindungen werden erfindungsgemäß mit einem aromatischen oder heteroaromatischen Edukt der Formel (III)

Ar²-Y (III),

worin Ar² die zuvor dargelegte Bedeutung hat und Y ein Halogen oder eine Gruppe ausgewählt aus Arylsulfonyloxy, Perfluoroalkylsulfonyloxy, Alkylcarbonyloxy, Arylcarbonyloxy, Alkyloxycarbonyloxy bzw. Aryloxycarbonyloxy darstellt, umgesetzt.

Die Gruppe Y des aromatischen oder heteroaromatischen Eduktes gemäß Formel (III) ist vorzugsweise ausgewählt aus der Reihe Chlor, Brom, Jod oder Gruppen aus der Reihe Arylsulfonyloxy, insbesondere para-Tolylsulfonyloxy; Alkylsulfonyloxy, insbesondere Methylsulfonyloxy (Mesylat) und Perfluoralkylsulfonyloxy, wie Perfluorbutylsulfonyloxy (Nonaflat), Trifluormethylsulfonyloxy (Triflat), Alkylcarbonyloxy, Arylcarbonyloxy, Alkyloxycarbonyloxy bzw. Aryloxycarbonyloxy. Hierbei werden insbesondere Edukte gemäß Formel (III) eingesetzt, die Brom, lod oder Perfluoralkylsulfonyloxy (insbesondere Triflat) als Gruppe Y aufweisen.

Gemäß einer bevorzugten Ausführungsform werden aromatische Edukte der Formel (IIIa) eingesetzt 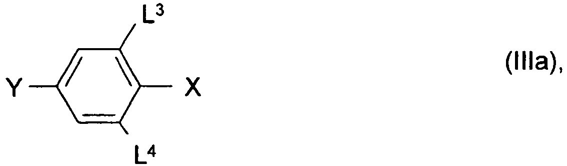 worin
- Y: Br, Cl, I oder ein Pseudohalogen, vorzugsweise Br, I oder Perfluoralkylsulfonyloxy,
- L³ und L⁴: unabhängig voneinander Wasserstoff oder Fluor,
- X: F, Cl, CN, SF₅, SCN, NCS, halogenierter Alkylrest, bevorzugt halogenierter Alkanylrest, halogenierter Alkenylrest, halogenierter Alkoxyrest oder halogenierter Alkenyloxyrest mit bis zu 6 C-Atomen,

bedeuten.

Katalysatoren zur Durchführung der erfindungsgemäßen Umsetzung sind allgemein bekann, wobei dieselben bevorzugt Palladium enthalten. Die bevorzugt einzusetzenden Katalysatoren können aus gängigen Palladium(II)-Salzen wie etwa Palladiumchlorid, -bromid, -iodid, -acetat, -acetylacetonat, die wahlweise durch weitere Liganden wie z. B. Alkylnitrile stabilisiert sein können, bzw. aus Pd (0)-Spezies wie Palladium auf Aktivkohle oder Tris(dibenzylidenaceton)dipalladium und Phosphinliganden PR₁R₂R₃, wobei Rᵢ für Substituenten aus der Reihe Wasserstoff, lineares und verzweigtes C₁-C₈ Alkyl, Vinyl-, Aryl-, oder Heteroaryl aus der Reihe Pyridin, Pyrimidin, Pyrrol, Thiophen, Furan stehen, die ihrerseits mit weiteren Substituenten aus der Reihe lineare und verzweigte C₁-C₈ Alkyl, vorzugsweise C₁-C₈ Alkanyl, oder C₁-C₈ Aryl, lineares und verzweigtes C₁-C₈ Alkyloxy oder C₁-C₈ Aryloxy, halogeniertes lineares und verzweigtes C₁-C₈ Alkyl vorzugsweise C₁-C₈ Alkanyl, oder halogeniertes C₁-Cₐ Aryl, lineares und verzweigtes C₁-C₈ Alkyl, vorzugsweise C₁-C₈ Alkanyl, oder C₁-C₈ Aryloxycarbonyl, lineares und verzweigtes C₁-C₈ Alkylamino, lineares und verzweigtes C₁-C₈ Dialkylamino, C₁-C₈ Arylamino, C₁-Cₐ Diarylamino, Formyl, Hydroxy, Carboxyl, Cyano, und Halogene wie F, Cl, Br und I substituiert sein können, in situ erzeugt werden. Alternativ dazu können auch definierte Palladiumkomplexe eingesetzt werden, die aus den oben genannten Liganden in einem oder mehreren Verfahrensschritten zuvor erzeugt wurden. Besonders bevorzugt kann insbesondere Bis-(triphenylphosphin)-palladium-(II)-chlorid eingesetzt werden.

Überraschende Vorteile können bei der Durchführung des erfindungsgemäßen Verfahrens durch Einsatz einer Katalysatormenge im Bereich von 0,001 mol% bis 20 mol%, bezogen auf das aromatische oder heteroaromatische Edukt der Formel (III), erzielt werden. Vorzugsweise wird eine Katalysatormenge von 0,01 mol% bis 3 mol% eingesetzt.

Das erfindungsgemäße Verfahren ist durch Verwendung von basischen Boraten, vorzugsweise Metaboraten (BO₂⁻, vor und nachstehend als Metaborat bezeichnet), Tetraboraten, Pentaboraten oder Borax als Base gekennzeichnet, wobei vorzugsweise die Alkalisalze (insbesondere Natriumsalze) eingesetzt werden (Alkaliborate), und besonders bevorzugt das Tetraborat NaBO₂·4H₂O (bzw. Na₂B₂O₄·8H₂O) verwendet wird. Diese Verbindung hat die CAS Nummer 10555-76-7. Alternativ eignet sich NaBO₂·2H₂O (CAS 16800-11-6). Weitere Alternativen beinhalten Na₂B₄O₇·5H₂O, K₂B₄O₇·4H₂O, KB₅O₈·4H₂O, NH₄B₅O₈-4H₂O oder Borax (Na₂B₄O₇·10H₂O).

Mit Vorteil wird das basische Borat, vorzugsweise das Metaborat im Überschuss in Bezug auf die aromatische oder heteroaromatische Borverbindung gemäß Formel (II) eingesetzt. Vorzugsweise beträgt das Molverhältnis von basischem Borat, insbesondere Metaborat zu Borverbindung gemäß Formel (II) mindestens 0,8:1, besonders bevorzugt mindestens 1,2:1. Besondere Vorteile können dadurch erzielt werden, dass das molare Verhältnis von basischem Borat, vorzugsweise Metaborat zu Borverbindung gemäß Formel (II) im Bereich von 1,1:1 bis 3:1, besonders bevorzugt 1,3:1 bis 2:1 liegt.

Das erfindungsgemäße Verfahren zeichnet weiterhin durch die Verwendung eines wasserarmen bzw. wasserfreien polaren Lösungsmittels aus. Vorzugsweise beträgt der Wassergehalt höchstens 1 Gew.-%, bezogen auf das Gesamtgewicht der Reaktionsmischung.
Polare Lösungsmittel sind aus dem Stand der Technik bekannt, wobei hierunter insbesondere Lösungsmittel zu verstehen sind, die das einzusetzende basische Borat, vorzugsweise das Metaborat in ausreichendem Maß lösen. Besonders bevorzugt beträgt die Löslichkeit des basischen Borats, vorzugsweise des Metaborats im polaren Lösungsmittel mindestesn 0,1 g/L, besonders bevorzugt mindestens 1 g/L bei 50°C. Erfindungsgemäß weist das polare Lösungsmittel eine Dielektrizitätskonstante εᵣ (25°C) >15 (d.h. größer als Isoamylalkohol), bevorzugt > 20 (d.h. größer als Isopropylalkohol) und besonders bevorzugt > 30 (d.h. größer als Benzonitril) auf. Weiterhin weist das polare Lösungsmittel alternativ eine Polaritätskonstante E_{T} (30) >175 kJ/mol (d.h. größer als Methylenchlorid), bevorzugt > 200 kJ/mol (d.h. größer als Isoamylalkohol) und besonders bevorzugt > 210 kJ/mol auf (d.h. größer als 1-Butanol), wobei diese Werte bei 30°C bestimmt werden. Dabei entsprechen hohe *E*_{T}(30)-Werte einer hohen Solvens-Polarität. Viele beispielhafte Werte können folgenden Artikeln entnommen worden :
C. Reichardt, Chem. Rev. 1994, 94, 2319 - 2358.
C. Reichardt, G. Schäfer, Liebigs Ann. 1995, 1579 - 1582.
R. Eberhardt, S. Löbbecke, B. Neidhart, C. Reichardt, Liebigs Ann. /Recueil 1997, 1195 - 1199.
C. Reichardt, Green Chem. 2005, 7, 339 - 351. Weiterhin kann die Polaritätskonstate gemäß C. Reichardt "Solvent Effects in Organic Chemistry" Weinheim : VCH, 2002. , ISBN-10: 3-527-30618-8 gemessen werden.

Zu den bevorzugt einzusetzenden polaren Lösungsmittel zählen unter anderem Alkohole, vorzugsweise Methanol, Ethanol, Propanol, Butanol und Pentanol; bevorzugt Methanol; polare aprotische Lösungsmittel, beispielsweise Dimethylformamid (DMF), Sulfolan, Ethylencarbonat und Propylencarbonat.

Überraschende Vorteile können insbesondere durch ein Lösungsmittelsystem erzielt werden, welches neben einem polaren zusätzlich ein hydrophobes Lösungsmittel umfasst.

Hydrophobe Lösungsmittel weisen bevorzugt eine Dielektrizitätskonstante εᵣ < 5,5, besonders bevorzugt < 3 und ganz besonders bevorzugt < 2,5 auf.

Weiterhin weist das hydrophobe Lösungsmittel bevorzugt eine Polaritätskonstante E_{T}(30) <150 kJ/mol (d.h. kleiner als 1,4-Dioxan), besonders bevorzugt < 145 kJ/mol (d.h. kleiner als Piperidin, z.B. Diethylether, Benzol, Toluol) und ganz besonders bevorzugt < 135 kJ/mol auf (d.h. kleiner als Tetrachlorkohlenstoff, z.B. Cyclohexan, Hexan, Pentan, allg. Petrolether).

Zu den bevorzugten hydrophoben Lösungsmitteln gehören aromatische Kohlenwasserstoffe, wie z.B. Benzol, Toluol und aliphatische Kohlenwasserstoffe, insbesondere Alkane und Cycloalkane. Besondere Vorteile können insbesondere durch die Verwendung von Alkanen und Cycloalkanen mit 5 bis 15 Kohlenstoffatomen erzielt werden, wobei n-Hexan, Cyclohexan, n-Heptan, Methylcyclohexan, Oktan, Dimethylcyclohexan und Nonan besonders bevorzugt sind.

Die Reaktionsmischung zur Durchführung des erfindungsgemäßen Verfahrens kann weitere Komponenten enthalten. Hierzu gehören unter anderem Reduktionsmittel, wie zum Beispiel Hydrazin , Triethylamin, bevorzugt Hydraziniumhydroxyd.

Das erfindungsgemäße Verfahren kann mit Vorteil bei Temperaturen im Bereich von -20 °C bis 150 °C, vorzugsweise bei 0 °C bis 80 °C und besonders bevorzugt bei 20 °C bis 60 °C durchgeführt werden.

Durch das vorliegende Verfahren werden sehr geringe Anteile an unerwünschten Nebenprodukten gebildet, ohne dass eine Verringerung der Ausbeuten eintritt. Demgemäß kann die Aufreinigung der Rohprodukte besonders einfach und kostengünstig ausgestaltet werden. Gemäß einer besonderen Ausführungsform der vorliegenden Erfindung genügt die Durchführung eines einzigen Kristallisationsvorgangs, vorzugsweise aus Ethanol zum Erhalt von Produkten mit einer Reinheit von bevorzugt mindestens 95%, insbesondere mindestens 99,0% besonders bevorzugt mindestens 99,5%. Der Gehalt an Dimer(Nebenprodukt) liegt dabei bevorzugt vor der Kristallisation (Rohprodukt) bei < 0,5%, besonders bevorzugt < 0,4%, nach der Kristallisation < 0,2%, besonders bevorzugt < 0, 1 %. Besondere Vorteile können insbesondere mit Lösungsmittelgemischen erzielt werden, die Heptan und Ethanol umfassen, wobei das Gewichtsverhältnis von Heptan zu Ethanol vorzugsweise im Bereich von 7:1 bis 1:2, besonders bevorzugt 3:1 bis 1:1 liegt.

Das Verfahren und die anschließende Aufarbeitung des Reaktionsgemisches kann grundsätzlich als Batch-Reaktion oder in kontinuierlicher Reaktionsweise durchgeführt werden. Die kontinuierliche Reaktionsweise umfasst z. B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Cokristallisation oder durch Nanofiltration an Membranen.

Weitere Kombinationen der Ausführungsformen und Varianten der Erfindung ergeben sich aus den Ansprüchen.

Weitere bevorzugte Verfahrensvarianten lassen sich den Beispielen entnehmen, deren Details - auch verallgemeinert nach allgemeiner Fachkenntnis - repräsentativ für bevorzugte Ausführungsformen des erfindungsgemäßen Verfahren und seiner Produkte sind.

Die Erfindung wird im Folgenden anhand von Ausführungsbeispielen näher erläutert, ohne dadurch jedoch beschränkt zu werden.

### Beispiele

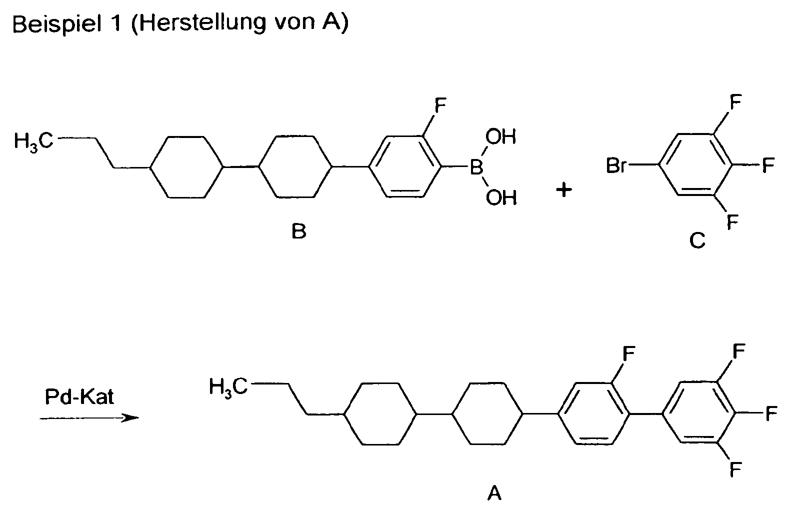

In einer 30 I Apparatur wurden unter Stickstoff 2,84 mol Verbindung B (C₂₁H₃₂BFO₂; M = 346,287 g/mol) in 4,1 kg n-Heptan vorgelegt. Zur erhaltenen Suspension wurden 2,98 mol Verbindung C (C₆H₂BrF₃; M = 210,979 g/mol), 4,26 mol Natriummetaborat Tetrahydrat (Na₂B₂O₄·8H₂O), 5,2 mmol Bis(triphenylphosphin)PdCl₂, 85,2 mmol Hydraziniumhydroxid und 6,2 kg Methanol gegeben. Die Mischung wurde auf 50°C erhitzt und 4 Stunden gerührt.

Nach Beendigung der Reaktion wurde die Phasen getrennt und die unpolare Phase mit 2,1 kg Wasser gewaschen. Nachfolgend wurde die Lösung mit 2,9 kg Heptan versetzt und die Wasserreste durch Destillation von 1,8 kg Lösungsmittel entfernt. Die erhaltene, von Wasser befreite Zusammensetzung (98 % Produkt; 0,3 % Dimer; HPLC und GC) wurde auf 50°C erhitzt und über etwa 3 kg Aluminiumoxid neutral filtriert. Der Filter wurde mit 3,8 kg Heptan gewaschen. Die erhaltene Zusammensetzung (>99 % Produkt; 0,04 % Dimer; HPLC) wurde durch Destillation von ca. 6,5 kg Heptan eingeengt, mit 2,4 kg Ethanol versetzt und auf ca. 70°C erhitzt, um eine Lösung zu erhalten. Die erhaltene Lösung wurde auf 0°C abgekühlt, um einen kristallinen Niederschlag zu erzeugen, der abgetrennt und mit Ethanol (700 g) gewaschen wurde.

Das Ergebnis einer gaschromatographischen Analyse zeigte, dass die Kristalle der erhaltenen Verbindung A (C₂₇H₃₂F₄; M = 346,287 g/mol) eine Reinheit von mindestens 99,5% aufwiesen, wobei der Anteil an Dimer etwa 0,08% betrug.

### Vergleichsbeispiel 1

Das Beispiel 1 wurde im Wesentlichen wiederholt, wobei jedoch im Syntheseschritt ein Lösungsmittelgemisch eingesetzt wurde, das Tetrahydrofuran und Wasser umfasste.

Das Ergebnis einer gaschromatographischen Analyse zeigte, dass die Kristalle der erhaltenen Verbindung A (C₂₇H₃₂F₄; M = 346,287 g/mol) eine Reinheit von ca. 99,0 % aufwiesen, wobei der Anteil an Dimer etwa 0,7% betrug.

## Patentansprüche

1. Verfahren zur Herstellung von aromatischen oder heteroaromatischen Verbindungen der Formel (I)
Ar¹-Ar² (I),
worin die Reste Ar¹ und Ar² jeweils unabhängig ein optional substituiertes aromatisches oder heteroaromatisches Ringsystem darstellen, umfassend die Umsetzung einer aromatischen oder heteroaromatischen Borverbindung der Formel (II) 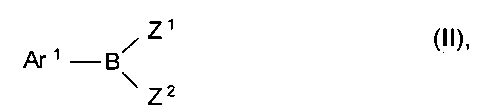 worin Ar¹ die zuvor genannte Bedeutung hat und die Reste Z¹ und Z² unabhängig Hydroxy, Dialkylamino, C₁ - C₁₅ Alkyloxy oder C₆ - C₁₅ Aryloxy darstellen, wobei die beiden Reste Z¹ und Z² auch ein Ringsystem bilden können, mit einem aromatischen Edukt der Formel (III)
Ar²-Y (III),
worin Ar² die zuvor dargelegte Bedeutung hat und Y ein Halogen, Arylsulfonyloxy, Alkylsulfonyloxy, Perfluoralkylsulfonyloxy, Alkylcarbonyloxy, Arylcarbonyloxy, Alkyloxycarbonyloxy oder Aryloxycarbonyloxy darstellt, unter Verwendung eines Lösungsmittels in Gegenwart einer Base,
**dadurch gekennzeichnet, dass** ein wasserarmes polares Lösungsmittel, das eine Polaritätskonstante E_{T} (30) > 175 kJ/mol und/oder eine Dielektrizitätskonstante εᵣ >15 aufweist, und ein Borat als Base zur Umsetzung eingesetzt werden, wobei der Wassergehalt der Reaktionsmischung höchstens 5 Gew.-% beträgt.

2. Verfahren nach Anspruch 1, **dadurch gekennzeichnet, dass** eine aromatische Borverbindung der Formel (IIa) 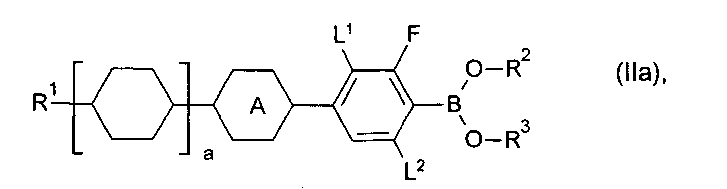 worin
R¹ einen Alkylrest mit 1 bis 15, vorzugsweise 1 bis 9 Kohlenstoffatomen, bevorzugt einen Alkanyl-, Alkenyl- oder Alkoxyrest mit bis zu 9 Kohlenstoffatomen, der substituiert oder unsubstituiert sein kann, bedeutet;
R² und R³ jeweils unabhängig Wasserstoff oder einen Alkylrest mit 1 bis 15, vorzugsweise 1 bis 9 Kohlenstoffatomen, bevorzugt einen Alkanyl- oder Alkenylrest, der substituiert oder unsubstituiert sein kann, wobei R² und R³ zusammen auch einen Rest -(CH₂)ₚ- mit p = 2 bis 4 oder 1,2-Phenylen bedeutet, wobei diese Reste optional ein- oder mehrfach durch n-Alkylreste mit 1-9 C-Atomen substituiert sein können, 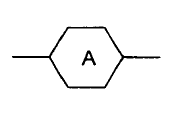 für 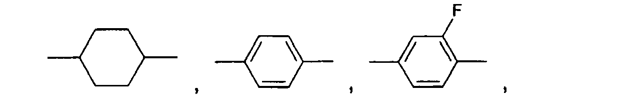 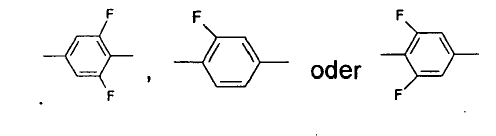 steht;
a 0 oder 1 bedeutet; und
L¹ und L² unabhängig voneinander Wasserstoff oder Fluor bedeuten,
eingesetzt wird.

3. Verfahren nach Anspruch 1 oder 2, **dadurch gekennzeichnet, dass** der Wassergehalt der Reaktionsmischung höchstens 1 Gew.-% beträgt.

4. Verfahren nach einem oder mehreren der Ansprüche 1 bis 3, **dadurch gekennzeichnet, dass** das polare Lösungsmittel eine Polaritätskonstante E_{T}(30) > 200 kJ/mol und/oder eine Dielektrizitätskonstante εᵣ > 20 aufweist.

5. Verfahren nach einem oder mehreren der Ansprüche 1 bis 4, **dadurch gekennzeichnet, dass** das polare Lösungsmittel mindestens einen Alkohol umfasst.

6. Verfahren nach einem oder mehreren der Ansprüche 1 bis 5, **dadurch gekennzeichnet, dass** die Umsetzung in einem Lösungsmittelsystem mit einem polaren und einem hydrophoben Lösungsmittel erfolgt.

7. Verfahren nach Anspruch 6, **dadurch gekennzeichnet, dass** das hydrophobe Lösungsmittel mindestens ein Alkan umfasst.

8. Verfahren nach einem oder mehreren der Ansprüche 1 bis 7, **dadurch gekennzeichnet, dass** das molare Verhältnis von Base zu Borverbindung der in Anspruch 1 dargelegten Formel (II) im Bereich von 1,3:1 bis 2:1 liegt.

9. Verfahren nach einem oder mehreren der Ansprüche 1 bis 8, **dadurch gekennzeichnet, dass** als Base ein Borat ausgewählt aus Natrium- oder Kaliumboraten eingesetzt wird.

10. Verfahren nach einem oder mehreren der Ansprüche 1 bis 9, **dadurch gekennzeichnet, dass** die Reaktion bei einer Temperatur im Bereich von 20 bis 60 °C durchgeführt wird.

## Claims

1. Process for the preparation of aromatic or heteroaromatic compounds of the formula (I)
Ar¹-Ar² (I),
in which the radicals Ar¹ and Ar² each independently represent an optionally substituted aromatic or heteroaromatic ring system, comprising the reaction of an aromatic or heteroaromatic boron compound of the formula (II) 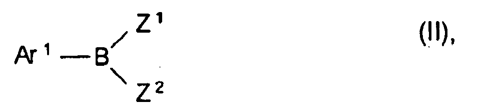 in which Ar¹ has the above-mentioned meaning and the radicals Z¹ and Z² independently represent hydroxyl, dialkylamino, C₁ - C₁₅ alkyloxy or C₆ - C₁₅ aryloxy, where the two radicals Z¹ and Z² may also form a ring system, with an aromatic starting material of the formula (III)
Ar²-Y (III),
in which Ar² has the above-mentioned meaning and Y represents a halogen, arylsulfonyloxy, alkylsulfonyloxy, perfluoroalkylsulfonyloxy, alkylcarbonyloxy, arylcarbonyloxy, alkyloxycarbonyloxy or aryloxycarbonyloxy, using a solvent in the presence of a base, **characterised in that** a polar solvent with a low water content which has a polarity constant E_{T}(30) > 175 kJ/mol and/or a dielectric constant εᵣ > 15, and a borate as base are employed for the reaction, where the water content of the reaction mixture is at most 5% by weight.

2. Process according to Claim 1, **characterised in that** an aromatic boron compound of the formula (IIa) 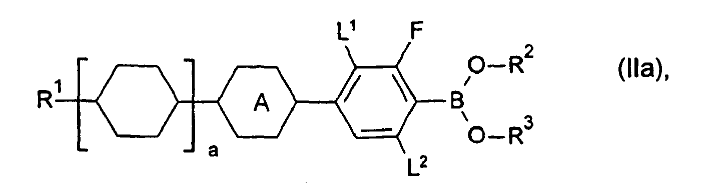 in which
R¹ denotes an alkyl radical having 1 to 15, preferably 1 to 9 carbon atoms, preferably an alkanyl, alkenyl or alkoxy radical having up to 9 carbon atoms, which may be substituted or unsubstituted;
R² and R³ each independently denote hydrogen or an alkyl radical having 1 to 15, preferably 1 to 9 carbon atoms, preferably an alkanyl or alkenyl radical, which may be substituted or unsubstituted, where R² and R³ together also denote a radical -(CH₂)ₚ-, where p = 2 to 4, or 1 ,2-phenylene, where these radicals may optionally be mono- or polysubstituted by n-alkyl radicals having 1-9 C atoms, 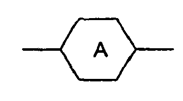 stands for 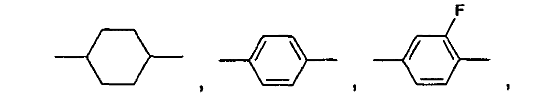 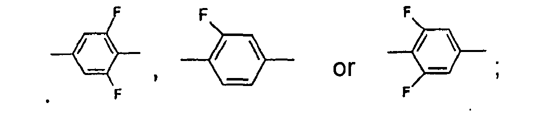
a denotes 0 or 1; and
L¹ and L², independently of one another, denote hydrogen or fluorine,
is employed.

3. Process according to Claim 1 or 2, **characterised in that** the water content of the reaction mixture is at most 1% by weight.

4. Process according to one or more of Claims 1 to 3, **characterised in that** the polar solvent has a polarity constant E_{T}(30) > 200 kJ/mol and/or a dielectric constant εᵣ > 20.

5. Process according to one or more of Claims 1 to 4, **characterised in that** the polar solvent comprises at least one alcohol.

6. Process according to one or more of Claims 1 to 5, **characterised in that** the reaction is carried out in the solvent system comprising a polar solvent and a hydrophobic solvent.

7. Process according to Claim 6, **characterised in that** the hydrophobic solvent comprises at least one alkane

8. Process according to one or more of Claims 1 to 7, **characterised in that** the molar ratio of base to boron compound of the formula (II) depicted in Claim 1 is in the range from 1.3:1 to 2:1.

9. Process according to one or more of Claims 1 to 8, **characterised in that** the base employed is a borate selected from sodium borate and potassium borate.

10. Process according to one or more of Claims 1 to 9, **characterised in that** the reaction is carried out at a temperature in the range from 20 to 60°C.

## Revendications

1. Procédé pour la préparation de composés aromatiques ou hétéroaromatiques de la formule (I)
Ar¹-Ar² (I),
dans laquelle les radicaux Ar¹ et Ar² représentent, chacun de manière indépendante, un système de cycle aromatique ou hétéroaromatique en option substitué, comprenant le fait de faire réagir un composé de bore aromatique ou hétéroaromatique de la formule (II) 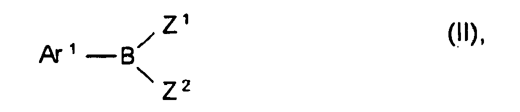 dans laquelle Ar¹ présente la signification mentionnée ci avant et les radicaux Z¹ et Z² représentent, de manière indépendante, hydroxyle, dialkylamino, C₁ - C₁₅ alkyloxy ou C₆ - C¹⁵ aryloxy, où les deux radicaux Z¹ et Z² peuvent également former un système de cycle, avec un matériau de départ aromatique de la formule (III)
Ar²-Y (III),
dans laquelle Ar² présente la signification mentionnée ci avant et Y représente un halogène, un arylsulfonyloxy, un alkylsulfonyloxy, un perfluoroalkylsulfonyloxy, un alkylcarbonyloxy, un arylcarbonyloxy, un alkyloxycarbonyloxy ou un aryloxycarbonyloxy, en utilisant un solvant en présence d'une base,
**caractérisé en ce qu'**un solvant polaire présentant une teneur en eau faible, lequel présente une polarité constante E_{T}(30) > 175 kJ/mol et/ou une constante diélectrique εᵣ > 15, et un borate en tant que base sont utilisés pour la réaction, où la teneur en eau du mélange de réaction est d'au plus 5% en poids.

2. Procédé selon la revendication 1, **caractérisé en ce qu'**un composé de bore aromatique de la formule (IIa) 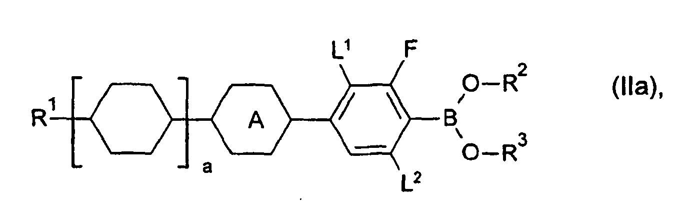 dans laquelle
R¹ représente un radical alkyle comportant de 1 à 15, de préférence de 1 à 9 atomes de carbone, de façon préférable un radical alkanyle, alkényle ou alcoxy comportant jusqu'à 9 atomes de carbone, lequel peut être substitué ou non substitué ;
R² et R³ représentent, chacun de manière indépendante, hydrogène ou un radical alkyle comportant de 1 à 15, de façon préférable de 1 à 9 atomes de carbone, de façon préférable un radical alkanyle ou alkényle, lequel peut être substitué ou non substitué, où R² et R³ représentent ensemble également un radical -(CH₂)ₚ-, où p = 2 à 4, ou 1 ,2-phénylène, où ces radicaux peuvent en option être mono- ou polysubstitués par des radicaux n-alkyle comportant 1-9 atomes de C, 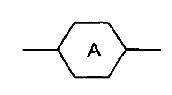 représente 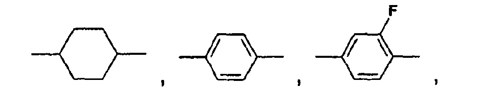 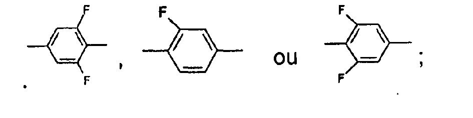
a représente 0 ou 1 ; et
L¹ et L² représentent, indépendamment l'un de l'autre, hydrogène ou fluor,
est utilisé.

3. Procédé selon la revendication 1 ou 2, **caractérisé en ce que** la teneur en eau du mélange de réaction est d'au plus 1% en poids.

4. Procédé selon une ou plusieurs des revendications 1 à 3, **caractérisé en ce que** le solvant polaire présente une constante de polarité ET(30) > 200 kJ/mol et/ou une constante diélectrique εᵣ > 20.

5. Procédé selon une ou plusieurs des revendications 1 à 4, **caractérisé en ce que** le solvant polaire comprend au moins un alcool.

6. Procédé selon une ou plusieurs des revendications 1 à 5, **caractérisé en ce que** la réaction est mise en oeuvre dans le système de solvants comprenant un solvant polaire et un solvant hydrophobe.

7. Procédé selon la revendication 6, **caractérisé en ce que** le solvant hydrophobe comprend au moins un alkane.

8. Procédé selon une ou plusieurs des revendications 1 à 7, **caractérisé en ce que** le rapport molaire base sur composé de bore de la formule (II) présentée selon la revendication 1 est dans la plage de 1,3:1 à 2:1.

9. Procédé selon une ou plusieurs des revendications 1 à 8, **caractérisé en ce que** la base utilisée est un borate choisi parmi le borate de sodium et le borate de potassium.

10. Procédé selon une ou plusieurs des revendications 1 à 9, **caractérisé en ce que** la réaction est mise en oeuvre à une température dans la plage de 20 à 60°C.
